# EUROPEAN PATENT APPLICATION

(11) **EP 1 813 271 A1**
(43) Date of publication of application: **01.08.2007**
(21) Application number: 05799945.0
(22) Date of filing: 26.10.2005
(51) Int. Cl.: A61K 31/198, A61P 1/00, A61P 1/06, A23L 1/305

(54) **PREVENTIVE/THERAPEUTIC AGENT FOR VISCERAL PAIN**

(30) Priority: 26.10.2004 JP 2004311090
(71) Applicant: Ajinomoto Co., Inc., Tokyo 104-8315 (JP)
(72) Inventor: YANO, Tetsuo, Ajinomoto Co., Inc., Kawasaki-shi, Kanagawa 2108681 (JP); HASHIMOTO, Masaki, Ajinomoto Co., Inc., Kawasaki-shi, Kanagawa 2108681 (JP); MIROSLAV, Smriga, Ajinomoto Co., Inc., Kawasaki-shi, Kanagawa 2108681 (JP); UNEYAMA, Hisayuki, Ajinomoto Co., Inc., Kawasaki-shi, Kanagawa 2108681 (JP); TORII, Kunio, Ajinomoto Co., Inc., Kawasaki-shi, Kanagawa 2108681 (JP)
(74) Representative: Nicholls, Kathryn Margaret
(86) International application number: PCT/JP2005/020051
(87) International publication number: WO 2006/046746

(57) **Abstract**

The present invention provides an agent for the prophylaxis or treatment of visceral pain associated with gastrointestinal tract diseases, which contains lysine. Using the prophylactic or therapeutic agent, gastrointestinal tract diseases accompanying visceral pain, such as intestinal disorder, irritable bowel syndrome, functional dyspepsia, gastro esophageal disease, functional abdominal pain syndrome, ulcerative colitis and Crohn's disease and the like can be treated or prevented. In addition, pharmaceutical agent, food and drink, and dietary supplement for the prophylaxis or treatment of visceral pain associated with such gastrointestinal tract diseases are provided. They are highly effective for visceral pain associated with gastrointestinal tract diseases as compared to existing pharmaceutical agents, show reduced side effects, and are highly effective.

## Description

### Technical Field

The present invention relates to an agent for the prophylaxis or treatment of visceral pain associated with gastrointestinal tract diseases. More particularly, the present invention relates to an agent for the prophylaxis or treatment of visceral pain, which contains lysine as an active ingredient. In addition, the present invention relates to a pharmaceutical agent, food and drink, and dietary supplement containing the prophylactic or therapeutic agent.

### Background Art

Gastrointestinal tract diseases such as non-organic disease, organic disease and the like (e.g., irritable bowel syndrome, functional dyspepsia, functional abdominal pain syndrome, gastro esophageal disease and IBD such as ulcerative colitis and Crohn's disease, and the like) accompany visceral pain, where visceral pain is one of the important symptoms in respective diseases (Rome II: The functional gastrointestinal disorders second edition, D.A. Drossman edit. (1995)). For the treatment of visceral pain, serotonin receptor antagonists, anticholinergic drugs, opioid agonists, antidepressants, antianxiety drugs, antispasmogenics and the like have been conventionally used. In recent years, NK₂ receptor antagonists have been clarified to suppress gastrointestinal motility and visceral pain, and developed as therapeutic drugs for gastrointestinal tract diseases, particularly irritable bowel syndrome (see Lecci, A., Maggi, C. A. Peripheral tacchykinin receptors as potential therapeutic targets in visceral diseases. Expert. Opin. Ther. Targets. 7: 343-362 (2003)) and functional dyspepsia (see Stanghellini, V., De Ponti, F., De Giorgio, R., Barbara, G., Tosetti, C., and Corinaldesi, R. New developments in the treatment of functional dyspepsia. Drug. 63: 869-892 (2003)). NK₂ receptors are expressed on the sensory nerve and the like. It is considered that inhibition of the functions of the receptor (neurotransmission and the like) by the antagonist affords expression of the efficacy. When baclofen, a GABA_{B} receptor agonist, was administered to patients with gastro esophageal disease, the pH in the esophagus was mostly maintained in the normal range and the symptoms were relieved (see Koek, GH., Sifrim, D., Lerut, T., Jamssens, J., Tack, J. Effect of GABA(B) agonist baclofen in patients with symptoms and duodeno-gastroesophageal reflux refractory to proton pump inhibitors. Gut. 52: 1397-1401 (2003)). As the mode of action thereof, it is considered that baclofen suppressed relaxation of the lower esophageal sphincter present in the esophagus and the cardiac region of the stomach, which in turn suppressed reflux of gastric acid into the esophagus. However, none of the drugs showed a sufficient effect, and a decisive and useful pharmaceutical agent is not available as the present situation. Under the circumstances, the development of a highly effective and highly safe pharmaceutical agent as an agent for the treatment or prophylaxis of visceral pain has been desired.

In the meantime, lysine has been clarified to have an anti-stress effect (see WO03/076445). In addition, lysine has been found to be a 5-HT₄ receptor antagonist (see Smriga, M., Torii, K. L-lysine acts like a partial serotonin receptor 4 antagonist and inhibits serotonin-mediated intestinal pathologies and anxiety in rats. Proc. Natl. Acad. Sci. U.S.A. 100: 15370-15375 (2003)). From the above, lysine has been suggested to influence the gastrointestinal motility. Moreover, the effectiveness of lysine on the stress has been investigated in a test using a rodent model animal and, for example, a treatment effect on a wrap stress rat model has been shown (see Japanese patent application No. 2002-271944). For these reasons, lysine is suggested to treat or prevent stress-related diarrhea and the like. However, the effect on the visceral pain has not been clear to date.

### Disclosure of the Invention

An object of the present invention is to provide an agent for the prophylaxis or treatment of visceral pain, which is more effective on visceral pain associated with gastrointestinal tract diseases than existing pharmaceutical agents, and shows reduced side effects. A further object is to provide a pharmaceutical agent, food and drink or a dietary supplement for the prophylaxis or treatment of visceral pain.

The present inventors have conducted intensive studies in an attempt to achieve the above-mentioned problems and found that administration or intake of lysine effectively improves visceral pain associated with gastrointestinal tract diseases, which resulted in the completion of the present invention.

Accordingly, the present invention provides the following.
(1) An agent for the prophylaxis or treatment of visceral pain associated with gastrointestinal tract diseases, which comprises lysine.
(2) The agent of (1), further comprising an amino acid other than lysine.
(3) The agent of (1) or (2), wherein the lysine is in an L-form.
(4) The agent of any one of (1) to (3), wherein the total daily dose of lysine is 1 - 30 g for an adult.
(5) The agent of any one of (1) to (4), wherein the gastrointestinal tract disease is a non-organic gastrointestinal tract disease.
(6) The agent of (5), wherein the non-organic gastrointestinal tract disease is an intestinal disorder.
(7) The agent of (6), wherein the intestinal disorder is at least one kind selected from the group consisting of irritable bowel syndrome, functional dyspepsia and functional abdominal pain syndrome.
(8) The agent of any one of (1) to (4), wherein the gastrointestinal tract disease is at least one kind of organic gastrointestinal tract disease selected from the group consisting of inflammatory bowel disease and gastro esophageal disease.
(9) A method for the prophylaxis or treatment of visceral pain associated with gastrointestinal tract diseases, which comprises administering an effective amount of lysine.
(10) The method of (9), further comprising administering an amino acid other than lysine.
(11) The method of (9) or (10), wherein the lysine is in an L-form.
(12) The method of any one of (9) to (11), wherein the daily dose of lysine for an adult is 1 - 30 g in total.
(13) The method of any one of (9) to (12), wherein the gastrointestinal tract disease is a non-organic gastrointestinal tract disease.
(14) The method of (13), wherein the non-organic gastrointestinal tract disease is an intestinal disorder.
(15) The method of (14), wherein the intestinal disorder is at least one kind selected from the group consisting of irritable bowel syndrome, functional dyspepsia and functional abdominal pain syndrome.
(16) The method of any one of (9) to (12), wherein the gastrointestinal tract disease is at least one kind selected from the group consisting of inflammatory bowel disease and gastro esophageal disease.
(17) Use of lysine for the production of an agent for the prophylaxis or treatment of visceral pain associated with gastrointestinal tract diseases.
(18) The use of (17), further comprising use of an amino acid other than lysine in combination.
(19) The use of (17) or (18), wherein the lysine is in an L-form.
(20) The use of any one of (17) to (19), wherein the total daily dose of lysine is 1 - 30 g for an adult.
(21) The use of any one of (17) to (20), wherein the gastrointestinal tract disease is a non-organic gastrointestinal tract disease.
(22) The use of (21), wherein the non-organic gastrointestinal tract disease is an intestinal disorder.
(23) The use of (22), wherein the intestinal disorder is at least one kind selected from the group consisting of irritable bowel syndrome, functional dyspepsia and functional abdominal pain syndrome.
(24) The use of any one of (17) to (20), wherein the gastrointestinal tract disease is at least one kind selected from the group consisting of inflammatory bowel disease and gastro esophageal disease.
(25) A pharmaceutical composition comprising lysine, which is used for the prophylaxis or treatment of visceral pain associated with gastrointestinal tract diseases.
(26) The pharmaceutical composition of (25), further comprising an amino acid other than lysine.
(27) The pharmaceutical composition of (25) or (26), wherein the lysine is an L-form.
(28) The pharmaceutical composition of any one of (25) to (27), wherein the total daily dose of lysine is 1 - 30 g for an adult.
(29) The pharmaceutical composition of any one of (25) to (28), wherein the gastrointestinal tract disease is a non-organic gastrointestinal tract disease.
(30) The pharmaceutical composition of (29), wherein the non-organic gastrointestinal tract disease is an intestinal disorder.
(31) The pharmaceutical composition of (30), wherein the intestinal disorder is at least one kind selected from the group consisting of irritable bowel syndrome, functional dyspepsia and functional abdominal pain syndrome.
(32) The pharmaceutical composition of any one of (25) to (28), wherein the gastrointestinal tract disease is at least one kind of organic gastrointestinal tract disease selected from the group consisting of inflammatory bowel disease and gastro esophageal disease.
(33) A pharmaceutical agent for the prophylaxis or treatment of visceral pain associated with gastrointestinal tract diseases, which comprises lysine.
(34) A food or drink comprising lysine, which is used for the prophylaxis or treatment of visceral pain associated with gastrointestinal tract diseases.
(35) The food or drink of (34) with an indication that the food or drink is used for the prophylaxis or treatment of visceral pain.
(36) A dietary supplement for the prophylaxis or treatment of visceral pain associated with gastrointestinal tract diseases, which comprises lysine.
(37) A commercial package comprising a pharmaceutical composition comprising lysine and a written matter comprising an explanation of the use of the composition for the prophylaxis and/or treatment of visceral pain associated with gastrointestinal tract diseases.
(38) A commercial package comprising an agent comprises lysine for the prophylaxis or treatment of visceral pain associated with gastrointestinal tract diseases, and a written matter comprising an explanation of use of the agent for the prophylaxis and/or treatment of visceral pain associated with gastrointestinal tract diseases.

### Brief Description of the Drawings

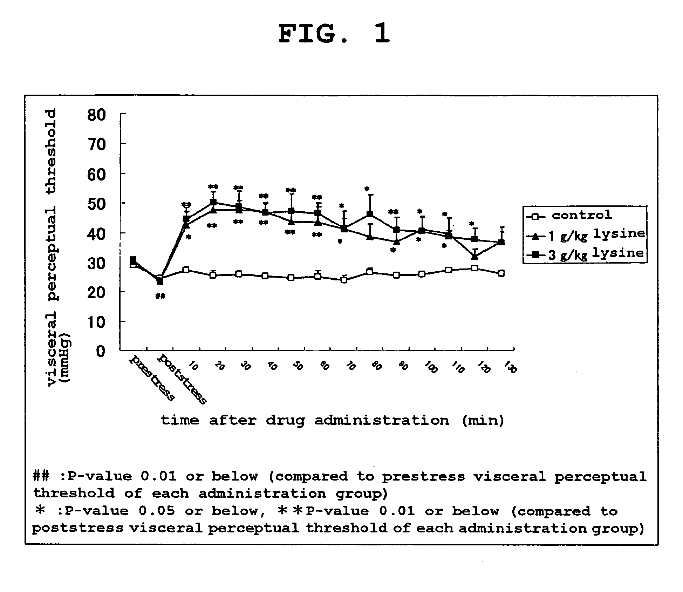

Fig. 1 is a graph showing the measurement of visceral perceptual threshold in stress induced colon distension visceral pain rat model.

### Best Mode for Embodying the Invention

The embodiment of the present invention is explained in the following.

The present invention is directed to an agent for the prophylaxis or treatment of visceral pain associated with gastrointestinal tract diseases, which is characterized in that lysine is contained as an active ingredient.

As to the isomer of lysine to be used as an active ingredient in the present invention, any of an L-form, D-form and DL-form can be used, with preference given to an L-form. The prophylactic or therapeutic agent of the present invention may further contain, where desired, an amino acid other than lysine (pharmaceutically active substance) within the range where the object of the present invention can be achieved. In other words, the prophylactic or therapeutic agent of the present invention can contain lysine together with an amino acid other than lysine (pharmaceutically active substance) in, for example, admixture or combination. As the amino acid other than lysine, essential amino acid and the like can be mentioned, and preferably, arginine, histidine, leucine, valine, glycine, aspartic acid, glutamic acid, alanine, glutamine, isoleucine and the like can be mentioned. Most preferably, lysine is used in admixture or combination with arginine, histidine, glycine, glutamic acid and the like. In this case, any mixtures or combinations of the objective active ingredient lysine in the present invention and an amino acid other than lysine (e.g., preferably arginine and the like) are encompassed in the present invention.

A part or the whole of lysine and other amino acids to be used as the active ingredient in the present invention may be not only in a free form but also in the form of a salt. In the present specification, lysine is a concept including a salt thereof. The salt form includes acid addition salt, salt with a base and the like. It is preferable to select a salt of lysine or the above-mentioned other amino acids, which is acceptable as a pharmaceutical product or food. As a substance that is added to lysine or the above-mentioned other amino acids to form a salt acceptable as a pharmaceutical product or food and drink, for example, inorganic salts with hydrogen chloride, hydrogen bromide, sulfuric acid, phosphate and the like, organic salts with acetic acid, lactic acid, citric acid, tartaric acid, maleic acid, fumaric acid, monomethylsulfuric acid and the like can be mentioned.

The prophylactic or therapeutic agent of the present invention is used for the treatment, prophylaxis and the like of visceral pain. The treatment here is a concept including improvement of symptoms, as well as prevention of the progression (deterioration) of medical conditions and symptoms.

In the present invention, the "visceral pain" can be defined to be induced by a substance released in the body by contraction or distension, inflammation of the gastrointestinal tract such as esophagus, stomach, small intestine (duodenum, jejunum, ileum) and large intestine (colon, rectal) and the like, during gastrointestinal tract diseases (Abdominal Pain, Sono Rinsho to Kiso, Revised Edition, 1987, ed. Fujiwara et al., Gendai-Iryo-Sha). The gastrointestinal tract disease in the present invention includes non-organic gastrointestinal tract diseases and organic gastrointestinal tract diseases. As the non-organic gastrointestinal tract diseases, intestinal disorder, for example, irritable bowel syndrome, functional dyspepsia and functional abdominal pain syndrome and the like can be mentioned. As the organic gastrointestinal tract diseases, for example, gastro esophageal disease and inflammatory bowel diseases (e.g., ulcerative colitis and Crohn's disease etc.) can be mentioned.

The therapeutic or prophylactic agent of the present invention is particularly effective for, of the visceral pains, gastrointestinal tract pain. The gastrointestinal tract pain refers to a pain in the esophagus, stomach, small intestine or large intestine.

The prophylactic or therapeutic agent of the present invention is useful for mammals (e.g., mouse, rat, hamster, rabbit, cat, dog, bovine, sheep, swine, human etc.).

The administration mode (or ingestion mode as food) of the pharmaceutical agent of the present invention is not particularly limited, and oral administration or parenteral administration (intake) such as instillation administration, injection administration (transvenous administration) and the like can be employed without particular limitation. Since the active ingredient is an amino acid, oral administration is preferable. The dosage form of the oral administration includes granule, fine granule, dusting powder, coated tablet, tablet, suppository, powder, (micro)capsule, chewable, syrup, juice, liquid, suspension, emulsion, and the like. For injection, general dosage forms of pharmaceutical preparations such as direct intravenous injection, instillation administration, preparation prolonging the release of activity substance and the like can be employed.

For oral administration, the dose varies depending on the symptom and age of patients to be the administration subject, and administration method. However, the daily dose is generally about 1 - 30 g, preferably about 1 - 20 g, more preferably about 1 - 10 g, for an adult based on lysine.

The dose for parenteral administration (intake) such as instillation administration, injection administration (transvenous administration) and the like can be about 1/10 to 1/20 of the preferable dose (intake) of the aforementioned oral administration.

The prophylactic or therapeutic agent of the present invention can be formulated according to a conventional method. When necessary for formulation, pharmacologically acceptable various substances (as auxiliaries) for preparations can be added. While the substance for preparation can be appropriately selected according to the dosage form of the preparation, it includes, for example, excipient, diluent, additive, disintegrant, binder, coating agent, lubricant, glidant, glazing agent, flavor, sweetener, solubilizer and the like. Specific examples of the substance for preparation include magnesium carbonate, titanium dioxide, lactose, mannitol and other saccharides, talc, milk protein, gelatin, starch, cellulose and derivatives thereof, animal and vegetable oil, polyethylene glycol, and solvent, such as sterile water and monovalent or polyvalent alcohol (e.g., glycerol and the like).

The prophylactic or therapeutic agent of the present invention can be formulated by a conventional method, as well as in the form of various pharmaceutical preparations to be developed in the future. A method to be developed in the future can be appropriately employed for the preparation.

Needless to say, the prophylactic or therapeutic agent of the present invention in the above-mentioned dosage form should contain the aforementioned component in an amount effective for affording the efficacy. That the aforementioned component should be contained in an amount effective for affording the efficacy also applies to the pharmaceutical agent, food and drink, and dietary supplement to be mentioned below.

The prophylactic or therapeutic agent of the present invention can be particularly used as a pharmaceutical agent, food and drink, or dietary supplement. When used as a pharmaceutical agent, the form thereof is not particularly limited and the above-mentioned preparation may be directly used. When used as a food and drink, or dietary supplement, the form thereof is not particularly limited and, for example, lysine alone may be added to food and drink, and dietary supplement in an amount necessary for exhibiting the effect of the present invention.

Since the active ingredient of the present invention is an amino acid, it is superior in the safety, and can be conveniently used in the form of a food or drink. There is no particular difficulty in applying the present invention to food and drink and, for example, it can be mixed with juice, milk, confectionery, jelly and the like and served. It is also possible to provide such food as a Food with Health Claims which includes food and drink, particularly Food for Specified Health Uses and the like, indicating use of the present invention for the prophylaxis or treatment of visceral pain, and the like.

For use of the pharmaceutical agent of the present invention as a dietary supplement, for example, it can be formed into tablet, capsule, powder, granule, suspension, chewable, syrup and the like. The dietary supplement in the present invention includes, in addition to those taken as food, those taken for the purpose of supplementing nutrition, which include nutritional supplement, supplement and the like. The dietary supplement in the present invention can also include a subset of food with health claims.

In a package comprising the composition or the pharmaceutical agent of the present invention and a written matter containing an explanation of the use thereof for its purpose, the written matter may be a package insert and the like containing an explanation relating to the use, efficacy, administration method and the like.

### Examples

The present invention The present invention is explained in more detail in the following by referring to Examples. The following Examples explain the present invention but do not limit the present invention.

### (Example 1)

### Effect of lysine on visceral pain in stress-induced colon distension rat model

As a wrap stress model, a model prepared according to the method reported by Williams et al. (Williams, C.L., Villar, R. G., Peterson, J. M., Burks, T. F. (1988) Stress-induced changes in intestinal transit in the rat: a model for irritable bowel syndrome. Gastroenterol. 94: 611-621) and, as a visceral pain model, a model prepared according to the method reported by Harada et al. (Harada, Y., Nishioka, K., Kitahata, L. M., Nakatani, K., and Collins, J. G. (1995) Contrasting actions of intrathecal U50,488H, morphine, or [D-Pen2, D-Pen5] enkephalin or intravenous U50,488H on the visceromotor response to colorectal distension in the rat. Anesthesiology. 83: 336-343) were used. That is, the forelimbs of 8-week-old male SD rat were wound with a cotton tape and left for 2 hr. Thereafter, under light ether anesthesia, the cotton tape was unwound, and a balloon catheter was noninvasively inserted from the anus of the rat. After 30 min, water was injected at a flow rate of 0.9 ml/min until the inner pressure of the distension balloon reached 80 mmHg (cut off value), and the inner pressure of the distension balloon at the time point when the contraction of the abdominal muscle was visually observed was measured as a visceral perceptual threshold. The interval of the balloon stimulation was 5 min, visceral perceptual threshold was measured 5 - 8 times, an average value of three stable and continuous threshold values was measured as an average visceral perceptual threshold. For statistical detection, the Student's t-test was used. At 5 min after the average visceral perceptual threshold measurement, 1 g/kg or 3 g/kg of lysine was orally administered, and the threshold was measured at 10 min intervals until 2 hr after the administration. For statistical detection of the effect of lysine, the Dunnett's test was used.

The results are shown in Fig. 1. The visceral perceptual threshold of the rat loaded with the stress was found to be 23.88 mmHg, and a significant reduction was confirmed as compared to the visceral perceptual threshold (30.17 mmHg) of the rat free of the stress.

When 1 g/kg or 3 g/kg of lysine was orally administered, moreover, the visceral perceptual threshold that decreased due to the stress significantly increased from 10 min after the administration, and a significant increase was observed for about 2 hr.

### (Example 2)

### Action of lysine on GABA_{B} receptor

A test for GABA_{B} receptor was performed according to the method reported by Kerr et al. (Kerr, D. I. B., Ong, J., Johnston, G. A. R., Abbenante, J. and Prager, R. H. (1988) 2-hydroxy-saclofen: an improved antagonist at central and peripheral GABAB receptors. Neurosci. Lett. 92: 92-96). The ileum was isolated from a guinea pig. The isolated tissue was hung in a solution of buffer (NaCl 118.0, KCl 4.7, MgSO₄ 1.2, CaCl₂ 2.5, KH₂PO₄1.2, NaHCO₃ 25, glucose 11.0 (pH 7.4) (unit:mM) 37°C) aerated with oxygen (95% oxygen, 5% carbon dioxide), added with naloxone (1 µM, opioid receptor antagonist), metisergide (1 µM, 5-HT₂ receptor antagonist), ondansetron (10 µM, 5-HT₃ receptor antagonist), GR113808 (0.01 µM, 5-HT₄ receptor antagonist) and indomethacin (1 µM, prostaglandin release suppressant). After 60 min or more, lysine (0.3, 3, 30 mM) was added to the buffer, and the influence on the contraction of the ileum by electric stimulation was measured.

The results are shown in Table 1. Lysine suppressed the ileum contraction via GABA_{B} receptor, in a dose-dependent manner.

**Table 1**

| compound | mM | contraction (%) |
|---|---|---|
| lysine | 0.3 | 0 |
| | 3.0 | -20 |
| | 30 | -77 |

In Table 1, the ileum contraction by electric stimulation was calculated as 100%.

### (Example 3)

### Action of lysine on NK₂ receptor

A test for NK₂ receptor was performed according to the method reported by Emonds et al. (Emonds-Alt, X., Advenier, C., Croco, T., Manara, L., Neliat, G., Poncelet, M., Proietto, V., Santucci, V., Soubrie, P., Van Broeck, D., Vilain, P., Le Fur, G. and Breliere, J. C. (1993) SR 48968, a neurokinin A (NK2) receptor antagonist. Regul. Peptides. 46: 31-36). The pulmonary artery was isolated from a rabbit. The isolated tissue was hung in a solution of buffer (NaCl 118.0, KC1 4.7, MgSO₄ 1.2, CaCl₂ 2.5, KH₂P0₄1.2, NaHCO₃ 25, glucose 11.0 (pH 7.4) (unit:mM) 37°C) aerated with oxygen (95% oxygen, 5% carbon dioxide), added with benexytramine (1 µM, α receptor antagonist), propranolol (1 µM, β receptor antagonist), pyrilamine (1 µM, histamine H1 receptor antagonist), atropine (1 µM, muscarinic receptor antagonist) and metisergide (1 µM, 5-HT₂ receptor antagonist). After 60 min or more, lysine (0.3, 3, 30 mM) was added to the buffer, and the contraction of the pulmonary artery due to [βAla⁸]NKA(4-10) was measured.

The results are shown in Table 2. Lysine suppressed the pulmonary artery contraction via NK₂ receptor, in a dose-dependent manner.

**Table 2**

| compound | mM | contraction (%) |
|---|---|---|
| lysine | 0.3 | 85 |
| | 3.0 | 41 |
| | 30 | 19 |
| [βAla⁸]NKA(4-10) | 3×10⁻⁴ | 100 |

In Table 2, the pulmonary artery contraction by [βAla⁸]NKA(4-10) was calculated as 100%.

From the foregoing, it is clear that the prophylactic or therapeutic agent provided by the present invention, which contains lysine, is effective for the treatment, prophylaxis and the like of visceral pain.

### Industrial Applicability

According to the present invention, an agent for the prophylaxis or treatment visceral pain associated with gastrointestinal tract diseases, particularly, visceral pain associated with gastrointestinal tract diseases, for example, non-organic gastrointestinal tract diseases such as intestinal disorders (irritable bowel syndrome, functional dyspepsia, functional abdominal pain syndrome etc.) and the like, organic gastrointestinal tract diseases such as inflammatory bowel diseases (ulcerative colitis and Crohn's disease etc.) and gastro esophageal disease and the like; and the like can be provided. The prophylactic or therapeutic agent of the present invention is superior in the safety, more useful as compared to existing gastrointestinal tract function improving drugs, and can be used as a pharmaceutical agent. Since the active ingredient is an amino acid, the agent is highly safe and almost free of side effects, and therefore, can be also used as food and drink or dietary supplement.

This application is based on a patent application No. 2004-311090 filed in Japan, the contents of which are incorporated in full herein by this reference.

## Claims

1. An agent for the prophylaxis or treatment of visceral pain associated with gastrointestinal tract diseases, which comprises lysine.

2. The agent of claim 1, further comprising an amino acid other than lysine.

3. The agent of claim 1 or 2, wherein the lysine is in an L-form.

4. The agent of any one of claims 1 to 3, wherein the total daily dose of lysine is 1 to 30 g for an adult.

5. The agent of any one of claims 1 to 4, wherein the gastrointestinal tract disease is a non-organic gastrointestinal tract disease.

6. The agent of claim 5, wherein the non-organic gastrointestinal tract disease is an intestinal disorder.

7. The agent of claim 6, wherein the intestinal disorder is at least one kind selected from the group consisting of irritable bowel syndrome, functional dyspepsia and functional abdominal pain syndrome.

8. The agent of any one of claims 1 to 4, wherein the gastrointestinal tract disease is at least one kind of organic gastrointestinal tract disease selected from the group consisting of inflammatory bowel disease and gastro esophageal disease.

9. A method for the prophylaxis or treatment of visceral pain associated with gastrointestinal tract diseases, which comprises administering an effective amount of lysine.

10. The method of claim 9, further comprising administering an amino acid other than lysine.

11. The method of claim 9 or 10, wherein the lysine is in an L-form.

12. The method of any one of claims 9 to 11, wherein the total daily dose of lysine is 1 - 30 g for an adult.

13. The method of any one of claims 9 to 12, wherein the gastrointestinal tract disease is a non-organic gastrointestinal tract disease.

14. The method of claim 13, wherein the non-organic gastrointestinal tract disease is an intestinal disorder.

15. The method of claim 14, wherein the intestinal disorder is at least one kind selected from the group consisting of irritable bowel syndrome, functional dyspepsia and functional abdominal pain syndrome.

16. The method of any one of claims 9 to 12, wherein the gastrointestinal tract disease is at least one kind selected from the group consisting of inflammatory bowel disease and gastro esophageal disease.

17. Use of lysine for the production of an agent for the prophylaxis or treatment of visceral pain associated with gastrointestinal tract diseases.

18. The use of claim 17, further comprising use of an amino acid other than lysine in combination.

19. The use of claim 17 or 18, wherein the lysine is in an L-form.

20. The use of any one of claims 17 to 19, wherein the total daily dose of lysine is 1 - 30 g for an adult.

21. The use of any one of claims 17 to 20, wherein the gastrointestinal tract disease is a non-organic gastrointestinal tract disease.

22. The use of claim 21, wherein the non-organic gastrointestinal tract disease is an intestinal disorder.

23. The use of claim 22, wherein the intestinal disorder is at least one kind selected from the group consisting of irritable bowel syndrome, functional dyspepsia and functional abdominal pain syndrome.

24. The use of any one of claims 17 to 20, wherein the gastrointestinal tract disease is at least one kind selected from the group consisting of inflammatory bowel disease and gastro esophageal disease.

25. A pharmaceutical composition comprising lysine, which is used for the prophylaxis or treatment of visceral pain associated with gastrointestinal tract diseases.

26. The pharmaceutical composition of claim 25, further comprising an amino acid other than lysine.

27. The pharmaceutical composition of claim 25 or 26, wherein the lysine is in an L-form.

28. The pharmaceutical composition of any one of claims 25 to 27, wherein the total daily dose of lysine is 1 - 30 g for an adult.

29. The pharmaceutical composition of any one of claims 25 to 28, wherein the gastrointestinal tract disease is a non-organic gastrointestinal tract disease.

30. The pharmaceutical composition of claim 29, wherein the non-organic gastrointestinal tract disease is an intestinal disorder.

31. The pharmaceutical composition of claim 30, wherein the intestinal disorder is at least one kind selected from the group consisting of irritable bowel syndrome, functional dyspepsia and functional abdominal pain syndrome.

32. The pharmaceutical composition of any one of claims 25 to 28, wherein the gastrointestinal tract disease is at least one kind of organic gastrointestinal tract disease selected from the group consisting of inflammatory bowel disease and gastro esophageal disease.

33. A pharmaceutical agent for the prophylaxis or treatment of visceral pain associated with gastrointestinal tract diseases, which comprises lysine.

34. A food or drink comprising lysine, which is used for the prophylaxis or treatment of visceral pain associated with gastrointestinal tract diseases.

35. The food or drink of claim 34 with an indication that the food or drink is used for the prophylaxis or treatment of visceral pain.

36. A dietary supplement for the prophylaxis or treatment of visceral pain associated with gastrointestinal tract diseases, which comprises lysine.

37. A commercial package comprising a pharmaceutical composition comprising lysine and a written matter comprising an explanation of the use of the composition for the prophylaxis and/or treatment of visceral pain associated with gastrointestinal tract diseases.
